Europäisches Patentamt

European Patent Office

Office européen des brevets

(11) Numéro de publication: **0 205 384 B1**

(19)

(12) **FASCICULE DE BREVET EUROPEEN**

(45) Date de publication du fascicule du brevet:
07.02.90

(21) Numéro de dépôt: 86401231.5

(22) Date de dépôt: 06.06.86

(51) Int. Cl.⁴: **A61N 1/06**, A61N 1/40, A61N 5/10, A61N 5/02

(54) **Dispositif de traitement par hyperthermie.**

(30) Priorité: 07.06.85 FR 8508641

(43) Date de publication de la demande:
17.12.86 Bulletin 86/51

(45) Mention de la délivrance du brevet:
07.02.90 Bulletin 90/6

(84) Etats contractants désignés:
DE IT SE

(56) Documents cités:
EP-A- 0 105 677
EP-A- 0 115 420
EP-A- 0 128 076
EP-A- 0 152 124
WO-A-81/03616
WO-A-85/02779
FR-A- 2 348 715
FR-A- 2 421 628
FR-A- 2 451 748
FR-E- 57 824
GB-A- 857 992
GB-A- 2 105 201
US-A- 4 346 715
US-A- 4 397 314
US-A- 4 448 198

(73) Titulaire: C.G.R. MeV, 551, route de la Minière,
F-78530 Buc(FR)

(72) Inventeur: Azam, Guy, THOMSON-CSF SCPI 19, avenue
de Messine, F-75008 Paris(FR)
Inventeur: Convert, Guy, THOMSON-CSF
SCPI 19, avenue de Messine, F-75008 Paris(FR)
Inventeur: Cosset, Jean-Marc, THOMSON-CSF
SCPI 19, avenue de Messine, F-75008 Paris(FR)
Inventeur: Dufour, Jacques, THOMSON-CSF
SCPI 19, avenue de Messine, F-75008 Paris(FR)
Inventeur: Mabire, Jean-Pierre, THOMSON-CSF
SCPI 19, avenue de Messine, F-75008 Paris(FR)

(74) Mandataire: Grynwald, Albert et al, THOMSON-CSF
SCPI, F-92045 PARIS LA DEFENSE CEDEX 67(FR)

## Description

L'invention concerne un dispositif de traitement par hyperthermie, permettant de chauffer une région malade d'un patient par la dissipation d'une énergie électrique appliquée selon un champ électrique grâce à des électrodes unipolaires disposées au voisinage de la région malade ou dans cette dernière. L'invention est remarquable en ce qu'elle permet de réduire considérablement les effets, traumatisants pour le patient, procurés par de tels traitements.

L'hyperthermie est un procédé connu, qui consiste à chauffer des tissus biologiques vivants à des températures sensiblement supérieures à leur température normale, et qui est utilisé dans le traitement de diverses maladies et notamment en cancérothérapie. Dans l'exemple de cette dernière application, il est souhaitable de chauffer les tissus à traiter à des températures de l'ordre de 44°C à 45°C, tout en évitant autant que possible, une élévation notable de la température des tissus sains environnants.

Le document US-A 4 448 198 décrit un appareil pour l'hyperthermie, comportant des sondes destinées à être reçues dans des tubes vecteurs destinés à être implantés à demeure dans la zone à traiter.

Cette condition soulève un problème qui réside dans la localisation correcte de la zone chauffée. Dans certaines configurations que peuvent présenter les tissus malades, cette localisation correcte du chauffage est impossible à obtenir avec des électrodes situées à l'extérieur du corps du patient. Aussi dans de nombreux cas, le traitement par hyperthermie est réalisé grâce à des électrodes implantées directement dans ou autour de la zone à traiter, de manière à mieux circonscrire la zone chauffée.

De telles sondes peuvent être soit du type bipolaire ou du type unipolaire. Dans le cas des sondes bipolaires, l'énergie électrique à haute fréquence délivrée par un générateur, sous la forme d'une tension, peut être appliquée à une unique sonde de ce type, pour chauffer la région dans laquelle elle est implantée. Dans le cas des sondes unipolaires, la tension à haute ou moyenne fréquence délivrée par le générateur est appliquée à deux sondes unipolaires distinctes, chacune des deux sondes étant reliée à l'un des pôles de sortie du générateur, la zone chauffée étant alors principalement établie entre les deux sondes unipolaires de type implantable.

L'implantation d'une sonde bipolaire ou unipolaire dans une région malade, représente pour un patient une opération qui peut s'accompagner d'une douleur non négligeable, et qui est particulièrement traumatisante en ce que les traitements par hyperthermie pour une zone à traiter donnée, doivent généralement être répétés à des fréquences variables et sur des périodes de temps comprises entre quelques jours et quelques dizaines de jours.

Un autre effet douloureux ou au moins très désagréable pour le patient, est lié à la particulière sensibilité des tissus cutanés à la température. En effet, l'expérience montre qu'il n'est pas possible en général pour le confort du patient, de maintenir, sans anesthésie, une température de l'ordre de 43° au niveau de la peau pendant des périodes de l'ordre d'une heure.

Une autre gêne importante pour le patient, durant le traitement, est liée à la rigidité mécanique des sondes notamment des sondes unipolaires. Ces dernières sont généralement constituées par des aiguilles métalliques, relativement rigides et qui peuvent, même après leurs positionnements, constituer la source d'une douleur ou d'une gêne plus particulièrement à la traversée de la peau.

Un but général de la présente invention est de réaliser une structure d'implantation des sondes, unipolaires ou bipolaires, permettant de diminuer autant que possible, les désagréments subis par le patient.

Plus précisément, un des buts de l'invention est de supprimer les désagréments dus à la répétition des implantations des sondes.

Un autre but de l'invention est de supprimer l'échauffement à la peau durant les traitements par hyperthermie des tissus sous-cutanés.

Enfin, un autre but de l'invention, est de supprimer ou atténuer la gêne apportée au niveau de la peau par la rigidité des sondes.

Il est à remarquer en outre que durant le traitement, certains tissus de la zone à traiter peuvent accuser des élévations de température différentes, ceci pouvant conduire au cours du traitement à modifier l'implantation des sondes. Aussi, un des buts de l'invention est également de permettre de modifier la configuration de la zone chauffée, sans exiger une modification de l'implantation des sondes.

Selon l'invention, un dispositif de traitement par hyperthermie comporte, au moins un générateur délivrant une énergie électrique alternative, ladite énergie électrique étant appliquée, selon un champ électrique, à une zone à traiter d'un patient grâce à des électrodes unipolaires reliées audit générateur, au moins une desdites électrodes unipolaires étant une sonde unipolaire implantable, et comporte en outre au moins un tube vecteur destiné à être implanté à demeure dans la zone à traiter en traversant la peau du patient et à contenir ladite sonde unipolaire durant une séance d'hyperthermie, ledit tube vecteur comportant au moins une extrémité tubulaire formée en un matériau isolant et prolongée par un tube métallique, ledit tube métallique étant destiné à contenir au moins partiellement ladite sonde unipolaire et à être en contact avec ladite zone à traiter, et ladite extrémité tubulaire étant destinée à être située au niveau de la peau, ladite sonde unipolaire étant introduite par ladite extrémité tubulaire dans ledit tube métallique avec lequel ladite sonde unipolaire est en contact électrique.

Nous entendons par l'expression "placée à demeure" en ce qui concerne le tube vecteur, une période de temps de, par exemple, plusieurs jours, à l'intérieur de laquelle plusieurs séances de traitement par hyperthermie sont prévues pour lesquelles la mise en place des électrodes ou sondes unipolaires s'effectue par leur introduction dans un tube vecteur.

Il est à remarquer qu'un avantage très important, apporté par l'invention, est de pouvoir associer facilement un traitement par curiethérapie au traitement par hyperthermie : les mêmes tubes vecteurs peuvent être utilisés :

a) - pour introduire des aiguilles radioactives (polonium ou autre) pour des séances de curiethérapie,

b) - pour introduire des sondes unipolaires, pour des séances d'hyperthermie.

L'invention sera mieux comprise grâce à la description qui suit faite à titre d'exemple non limitatif, et à l'aide des quatres figures annexées, parmi lesquelles :

- la figure 1 illustre l'implantation de sondes unipolaires dans une zone à traiter, avec une première version d'un dispositif de traitement par hyperthermie selon l'invention ;
- la figure 2 montre des détails d'un tube vecteur destiné, dans l'invention, à contenir une sonde unipolaire ;
- la figure 3 montre schématiquement une seconde version, à plusieurs générateurs, d'un dispositif de traitement selon l'invention permettant aisément de modifier la distribution des courants dans une zone à traiter ;
- la figure 4 illustre schématiquement une modification de la distribution des courants par rapport à la figure 3.

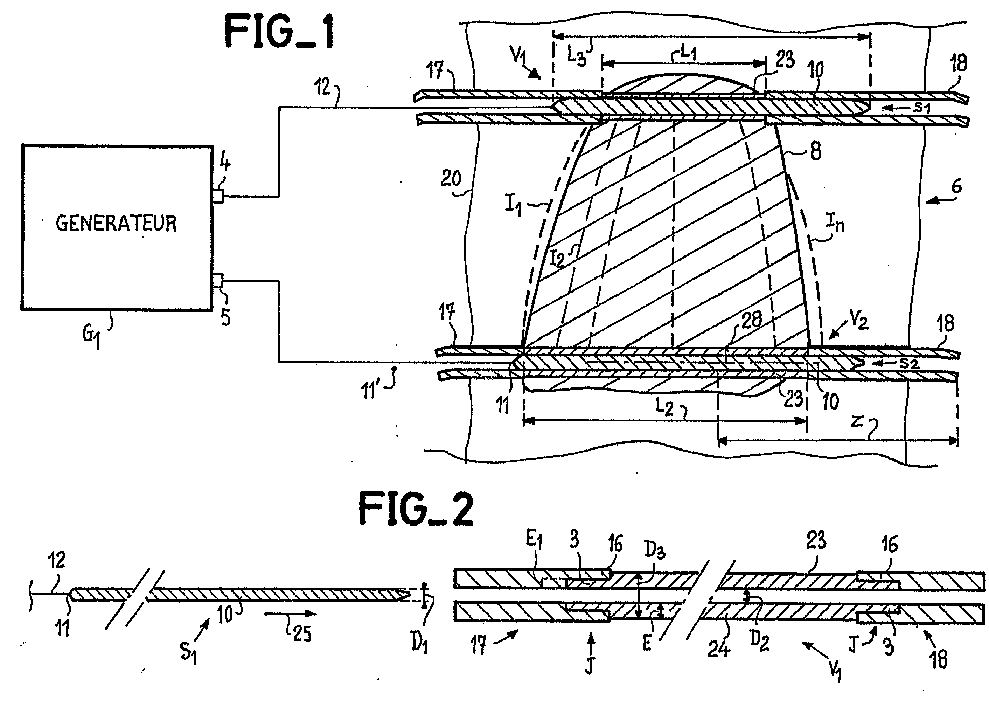

La figure 1 illustre un dispositif de traitement 1 par l'hyperthermie selon l'invention, et un exemple d'implantation de sondes unipolaires S1, S2.

Le dispositif de traitement comporte un générateur G1 du type fonctionnant à moyenne ou haute fréquence (de l'ordre de 100 KHZ à plusieurs MHZ), et de puissance moyenne (de l'ordre de quelques dizaines de Watt à quelques centaines de Watt). Le générateur G1 délivre, entre une première et une seconde bornes de sortie 4, 5, une énergie électrique alternative destinée à être dissipée dans le corps 6 (partiellement représenté) d'un patient, afin d'élever la température d'une zone 8 à traiter par hyperthermie. L'énergie électrique délivrée par le générateur G1, sous la forme d'une tension (non représentée) par example, est appliquée aux sondes unipolaires S1, S2, la première sonde unipolaire S1 étant à cet effet reliée à la première borne de sortie 4 et la seconde sonde unipolaire S2 étant reliée à la seconde borne de sortie 5. Dans l'exemple non limitatif décrit, les sondes unipolaires S1, S2 sont toutes deux du type implantables, c'est-à-dire constituées par une aiguille métallique 10 dont une extrémité 11 est connectée à un fil conducteur électrique 12 classique, par lequel elles sont reliées au générateur G1 ; mais il est également connu dans l'art antérieur, et ceci peut également être appliqué dans le cadre de l'invention, d'utiliser une unique sonde implantable S1, S2 du type unipolaire coopérant avec une électrode unipolaire (non représentée) destinée à être placée à l'extérieur du corps 6 du patient.

Dans l'art antérieur, ainsi qu'il a été expliqué dans le préambule, des sondes unipolaires implantables sont implantées directement dans les tissus à traiter à chaque séance d'hyperthermie, contrairement aux possibilités offertes par la présente invention.

En effet, selon une caractéristique de l'invention, le dispositif de traitement 1 comporte en outre des tubes vecteurs V1, V2 prévus pour être implantés dans la zone 8 à traiter ou à son voisinage, ainsi qu'il est représenté dans l'exemple non limitatif de la figure 1, et à y être laissés à demeure, c'est-à-dire sur une période de temps contenant plusieurs séances de traitement par l'hyperthermie. Les tubes vecteurs V1, V2 sont destinés à contenir, durant chaque séance de traitement, une sonde unipolaire S1, S2 introduite dans un tube vecteur V1, V2 par une première ou une seconde extrémité 17, 18 de ce dernier, au moins une de ces extrémités 17, 18 devant à cet effet être à l'extérieur du corps 6 du patient c'est-à-dire en dépassement par rapport à la peau 20 du patient.

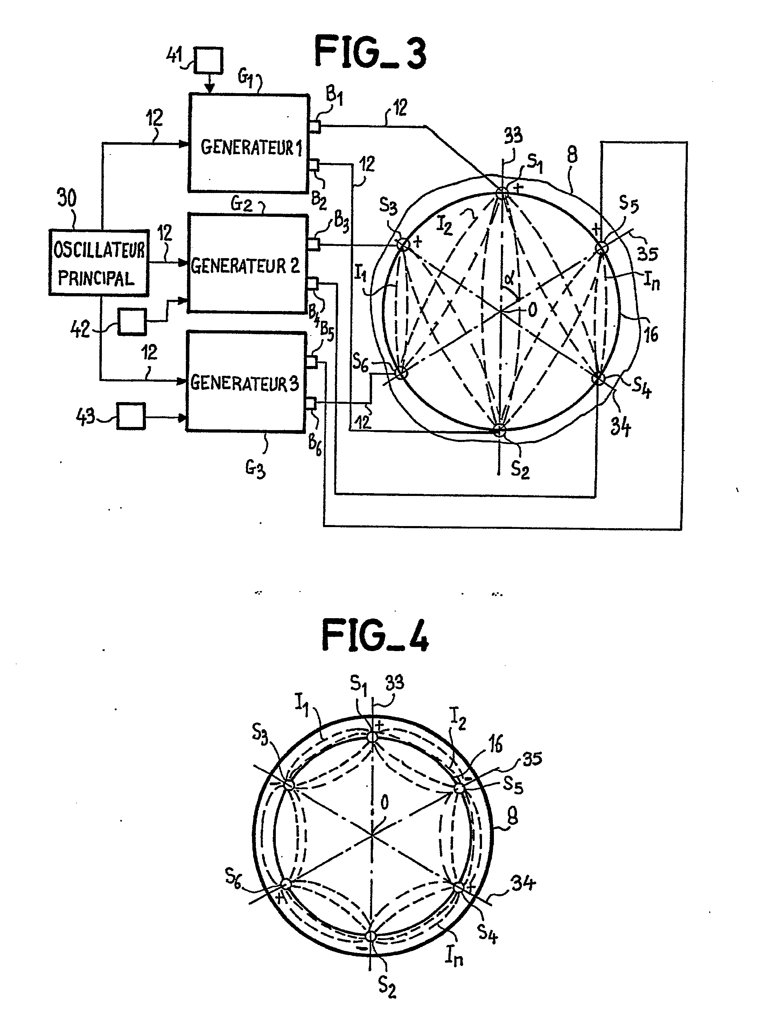

La figure 2 montre de manière plus claire, à titre d'exemple non limitatif, un tube vecteur V1 et une sonde unipolaire S1. Les première et seconde extrémités 17, 18 du tube vecteur V1, dont au moins une est nécessaire, sont réalisées, selon une caractéristique de l'invention, en un matériau électriquement isolant et, selon une autre caractéristique de l'invention, ce matériau doit en outre être souple ; ces caractéristiques étant chacune utile au confort du patient ainsi qu'il sera expliqué dans la suite de la description ; ces conditions étant remplies en utilisant des matériaux, tel que par exemple un polyamide connu sous la marque "nylon", ou en polytétrafluoroéthylène connu sous le nom de "Téflon"®. La première extrémité tubulaire 17 est prolongée par un tube métallique 23, communiquant avec l'extrémité tubulaire 17, de manière qu'une sonde unipolaire S1 puisse être introduite dans le sens montré par la première flèche 25 par exemple, jusque dans le tube métallique 23. Dans l'exemple non limitatif décrit, l'aiguille métallique 10 de la sonde unipolaire S1 a un diamètre D1 sensiblement inférieur à un diamètre intérieur D2 du tube métallique 23 ; ceci permettant d'assurer de manière simple, un contact électrique entre l'aiguille métallique 10 et le tube métallique 23 quand l'aiguille est introduite au moins partiellement dans ce dernier. En supposant que l'aiguille 10 ait de manière classique un diamètre D1 de l'ordre de 0,8 mm, et que le diamètre intérieur D2 du tube métallique 23 soit de l'ordre de 0,9 à 1 mm par exemple, afin de permettre le passage de l'aiguille 10 et assurer le contact électrique, et que d'autre part la paroi 24 du tube métallique 23 comporte une épaisseur E de l'ordre de 0,1 à 0,15 mm, un diamètre extérieur D3 du tube métallique 23 est de l'ordre de 1,1 à 1,2 mm. L'assemblage entre le tube métallique 23 et les extrémités tubulaires 17, 18 peut être réalisé par l'homme du métier de différentes manières, comme par exemple représenté à la figure 2, où le tube métallique 23 comporte du côté de sa jonction J avec les extrémités tubulaires 17, 18, une partie d'extrémité 3 où la paroi 24 comporte une épaisseur E1 plus faible, de manière que l'extrémité tubulaire 17, puisse être enfoncée sur cette partie d'extrémité 3 sans apporter de surépaisseur notable par rapport au diamètre extérieur D3 ; la partie d'extrémité 3 comportant ainsi un surgainage 16 isolant électriquement.

L'implantation du tube vecteur V1 dans la zone 8 à traiter, peut s'effectuer en utilisant une aiguille creuse (non représentée) du type appelé gouttière vectrice par exemple dont l'emploi dans le domaine médical est courant, notamment pour l'implatation de diverses sondes dans des tissus vivants ; une telle gouttière vectrice étant également décrite dans un brevet français publiés sous le n° 2 421 628. Le tube vecteur V1 peut être placé dans la gouttière vectrice, laquelle est ensuite introduite dans la zone 8 à traiter à l'emplacement voulu ; la gouttière vectrice est ensuite retirée avec précaution en faisant coulisser celle-ci sur le tube vecteur V1 qui reste en place.

En référence à nouveau à la figure 1, les tubes vecteurs V1, V2 étant en place, chaque sonde unipolaire S1, S2 est introduite dans un tube vecteur V1, V2. Les sondes unipolaires S1, S2 étant reliées au générateur 7, et celui-ci étant en fonctionnement, un champ électrique représenté sur la figure 1 par des lignes de champ $l_1$, $l_2$, ..., $l_n$ est établi entre les sondes unipolaires S1, S2 par l'intermédiaire du tube vecteur V1, V2, afin d'amener une élévation de la température de la zone à traiter 8 soumise au champ électrique. Un avantage de cette disposition réside en ce qu'elle permet de conférer aux tubes vecteurs V1, V2 des longueurs actives respectivement L1, L2 entre lesquelles est établi un champ électrique, indépendamment des longueurs L3 des sondes unipolaires S1, S2 ; ceci permettant de mieux circonscrire la zone 8 soumise au champ électrique. En effet, pour des fréquences inférieures à quelques MHZ, des épaisseurs de matériaux isolants de l'ordre du dixième de mm suffisent à empêcher l'établissement du courant électrique. Dans l'exemple non limitatif décrit, les longueurs actives L1, L2 correspondent à la longueur des tubes métalliques 23 mis à nu, c'est-à-dire non surgainés par les extrémités tubulaires 17, 18 en matériau isolant. Ceci permet notamment de conférer aux longueurs actives L1, L2 des dimensions éventuellement difféférentes, compatibles avec la géométrie de la zone 8 à traiter, tout en utilisant des sondes unipolaires S1, S2 ayant des aiguilles métalliques 10 d'une même longueur L3.

Les extrémités tubulaires 17, 18 assurent en outre un rôle de protection vis à vis de la peau 20, laquelle est particulièrement sensible aux élévations de température. Ainsi par exemple, dans le cas où une sonde unipolaire, la seconde sonde unipolaire S2 par exemple se trouve au niveau de la peau 20, n'étant pas par exemple entièrement enfoncée dans le second tube vecteur V2 : l'extrémité 11, de l'aiguille 10 connectée au fil 12, étant alors située à l'extérieur du corps 6 du patient en un point repéré 11' par exemple, la présence de l'extrémité tubulaire 17 empêche l'établissement du champ électrique au niveau de la peau 20 et évite ainsi la sensation de brûlures ressentie par le patient dans un tel cas. Cette possibilité de retrait d'une sonde unipolaire S1, S2 est d'autant plus importante, qu'elle permet de libérer dans le tube vecteur V1, V2 une zone Z opposée à la première extrémité 17, dans laquelle peut être introduite par la seconde extrémité 18, une aiguille d'un matériau radioactif (non représentée) d'un type classique. Ceci permet de réaliser de manière simultanée, un traitement par hyperthermie et un traitement par curiethérapie.

Un autre avantage très important pour le confort du patient, est apporté par la souplesse des extrémités tubulaires 17, 18, qui leur permet des mouvements relatifs par rapport à un axe longitudinal 28 des sondes unipolaires S1, S2, cette souplesse leur permettant de suivre des mouvements de la peau dus par exemple à de légers mouvements du patient, permettant ainsi d'éviter à ce dernier de ressentir des tiraillements douloureux.

Dans l'exemple non limitatif de la figure 1, deux tubes vecteurs V1, V2 ont été représentés, mais un plus grand nombre n de ces tubes vecteurs V1, V2, ..., Vn peuvent être implantés à demeure, dans le cas par exemple ou un plus grand nombre de sondes unipolaires S1, S2 sont utilisées avec par exemple plusieurs générateurs (non représentés sur la figure 1) ou encore dans le cas où le praticien désire modifier la configuration du chauffage d'une zone 8 traitée. Une telle modification peut être souhaitable d'une séance de traitement par hyperthermie à une séance suivante, ou même au cours d'une même séance, dans des cas par exemple où des tissus (non représentés) d'une zone à traiter 8 ont des sensibilités différentes au champ électrique.

La figure 3 illustre schématiquement une deuxième version du dispositif du traitement 1 selon l'invention, qui permet de réaliser des modifications de la zone chauffée ainsi que ci-dessus mentionnées, sans exiger la modification de position d'implantation de tubes vecteurs V1, V2, ... déjà implantés (non représentés sur la figure 3 pour plus de clarté). Il est à remarquer que cette version de l'invention est également applicable dans le cas d'une utilistion classique de sondes unipolaires implantables, c'est-à-dire sans utiliser les tubes vecteurs V1, V2, ..., V3.

Dans cette seconde version, le dispositif de traitement 1 doit comporter, en plus du premier générateur G1, au moins un générateur supplémentaire G2, c'est-à-dire au moins deux générateurs G1, G2 synchronisés en fréquence.

Aussi, dans l'exemple non limitatif décrit, le dispositif de traitement 1 comporte, outre le premier générateur G1, un second et un troisième générateur G2, G3, sensiblement d'un même type que le premier générateur G1 ; un nombre N de générateur, supérieur à 3 pouvant également être utilisés. Dans l'exemple non limitatif décrit, le dispositif de traitement 1 comporte en outre un oscillateur principal 30 relié par des liaisons 31 à chacun des générateurs G1, G2, G3 afin que ces derniers fonctionnent à une même fréquence F1 et selon une même phase.

La zone à traiter 8 est représentée sur la figure 3 par une vue en coupe selon un plan perpendiculaire à l'axe longitudinal 28 montré sur la figure 1. Dans l'exemple décrit, six sondes unipolaires S1, S2, ..., S6 sont, soit implantées directement dans la zone à traiter 8, soit placées chacune, ainsi que dans l'exemple précédent, dans un tube vecteur préalablement implanté ; les sonde unipolaires S1, ..., S6 étant montrées selon leur section, elles sont représentées sur la figure 3 selon des cercles. Dans l'exemple non limitatif décrit, les sondes unipolaires S1, ..., S6 sont disposées dans la zone 8 à traiter selon sensiblement un cercle 16 ayant un centre O, par lequel passe trois axes 33, 34, 35 formant entre eux un angle $\alpha$ d'environ 30° ; deux sondes unipolaires S1, ..., S6 reliées à un même générateur G1, G2, G3 étant, dans l'exemple non limitatif décrit, disposées sur un même axe 33, 34, 35, mais de manière opposée par rapport au centre O. Chacune des sondes S1, ..., S6 est reliée par un conducteur électrique 12 à une des bornes de sortie B1, B2, ..., B6 d'un générateur G1, G2, G3 :

- les première et seconde sondes S1, S2, disposées sur le premier axe 33 sont reliées respectivement à la première et à la seconde borne B1, B2 du premier générateur G1 ;
- les troisième et quatrième sondes S3, S4 disposées selon le second axe 34, sont reliées respectivement à une troisième et quatrième bornes de sorties B3, B4 du second générateur G2 ;
- les cinquième et sixième sondes S5, S6 disposées sur le troisième axe 35, sont reliées respectivement à une cinquième et une sixième borne de sortie B5, B6 du troisième générateur G3.

Les trois générateurs G1, G2, G3 fonctionnant à une même fréquence F1, si on repère les polarités +, - (pour un instant donné) des bornes de sorties B1, B2, et B3, B4 et B5, B6 correspondant à chacun des générateurs G1, G2, G3, on peut placer les sondes unipolaires S1 à S6 correspondantes à une position voulue dans la zone 8 à traiter, telle que par exemple montrée à la figure 3, de manière à établir le champ électrique entre ces sondes unipolaires S1 à S6 selon une configuration souhaitée ; ce positionnement des sondes pouvant s'effectuer par implantation directe ou en plaçant chaque sonde dans un tube vecteur comme il a été précédemment expliqué.

Ainsi à supposer que les première, troisième et cinquième bornes de sortie B1, B3, B5 aient une polarité positive +, les seconde, quatrième et sixième bornes de sortie B2, B4, B6 auront une polarité négative -, ces polarités +, - se retrouvant respectivement au niveau des sondes unipolaires S1, S3, S5 et S2, S4, S6.

Le champ électrique représenté par les lignes de champ $I_1$ à $I_n$, sera dans ces conditions établi dans l'ensemble de la zone 8 à traiter.

Ainsi qu'il a déjà été mentionné, il peut être souhaitable de modifier la distribution du champ électrique c'est-à-dire la configuration de la zone chauffée. Ceci est possible dans l'invention, sans causer de désagréments supplémentaires au patient, soit en substituant les sondes unipolaires S1, ..., S6 entre elles, par rapport aux tubes vecteurs, dans le cas où ceux-ci sont utilisés, soit en modifiant la polarité +, - respective des sondes S1, à S6, en agissant au niveau des générateurs G1, G2, G3, aussi bien dans le cas où les sondes unipolaires S1 à S6 sont implantées directement dans la zone 8 à traiter, que dans le cas où elles sont disposées dans des tubes vecteurs. A cet effet, chacun des générateurs G1, G2, G3 comporte un dispositif inverseur respectivement 41, 42, 43, permettant d'inverser la polarité entre deux bornes de sortie B1, B2 et B3, B4 et B5, B6 d'un même générateur G1, G2, G3. Les dispositifs de commutation 41, 42, 43 peuvent consister en des moyens de commutation classiques permettant par exemple pour le premier générateur G1, d'inverser des connexions (non représentées) établies entre les bornes de sortie B1, B2 et des amplificateurs (non représentés) que comporte de manière classique chaque générateur G1, G2, G3 ; une telle inversion des polarités étant réalisable de différentes manières, toutes à la portée de l'homme du métier, et peut consister également dans une inversion des fils conducteurs 12 connectés aux bornes de sortie B1, B2.

On peut ainsi, par la modification de la polarité des sondes S1 à S6, obtenir une modification de la distribution du champ électrique, telle que par exemple représentée à la figure 4.

La figure 4 représente la zone 8 à traiter selon une vue semblable à celle de la figure 3, c'est-à-dire selon un plan perpendiculaire l'axe longitudinal 28 montré sur la figure 1 ; les sondes unipolaires S1 à S6 occupent une même position que sur la figure 3, mais comportent des polarités +, - différentes, de manière que ces polarités soient successivement positives + et négatives -. Le champ électrique, représenté par les lignes de champ $I_1$, ..., $I_n$ établies entre des sondes adjacentes de polarité +, - opposées, correspond alors sensiblement à une distribution circulaire le long du cercle 16, c'est-à-dire autour du centre O qui dans cette configuration n'est pas soumis au champ électrique.

D'autres configurations du chauffage de la zone 8 à traiter peuvent être obtenues, en modifiant soit la distribution des sondes S1, ..., S6 dans les tubes vecteurs V1, V2, ..., soit en modifiant la polarité positive + ou négaive - conférées à chacune des sondes.

Cette description constitue un exemple non limitatif d'un dispositif de traitement 1 par hyperthermie selon l'invention, dans lequel n tubes vecteurs V1, V2, ..., Vn susceptibles d'être implantés à demeure permettent de supprimer les causes de l'inconfort ressenti par un patient avec des appareils de traitement selon l'art antérieur ; un dispositif de traitement 1 selon l'invention permettant en outre de réaliser un chauffage approprié à la zone 8 à traiter, avec beaucoup plus de souplesse que dans l'art antérieur.

**Revendications**

1. Dispositif de traitement par hyperthermie, comportant un générateur (G1) délivrant une énergie électrique alternative, ladite énergie électrique étant appliquée, selon un champ électrique ($I_1$, ..., $I_n$), à une zone (8) à traiter d'un patient grâce à deux électrodes unipolaires (S1, S2) reliées audit générateur (G1), au moins une desdites électrodes unipolaires (S1, S2) étant du type sonde unipolaire implantable, et comportant en outre au moins un tube vecteur (V1) destiné à être implanté à demeure dans la zone (8) à traiter, et à contenir une desdites sondes unipolaires (S1, S2) durant une séance de traitement, ledit tube vecteur (V1) comportant au moins une extrémité tubulaire ouverte (17, 18), formée en un matériau électriquement isolant, et prolongée par un tube métallique (23), ledit tube métallique (23) étant destiné à être au contact de la zone (8) à traiter et à contenir au moins partiellement ladite sonde unipolaire (S1, S2), ladite extrémité tubulaire (17, 18) étant destinée à être située au niveau de la peau, ladite sonde unipolaire (S1, S2) étant introduite par ladite extrémité tubulaire (17, 18) dans ledit tube métallique (23) avec lequel elle est mise en contact électrique.

2. Dispositif de traitement selon la revendication 1, caractérisé en ce que lesdites extrémités tubulaires (17, 18) sont réalisées en un matériau souple.

3. Dispositif de traitement selon la revendication précédente, caractérisé en ce que lesdites extrémités tubulaires (17, 18) sont réalisées en polytétrafluoroéthylène.

4. Dispositif de traitement selon l'une des revendications précédentes, caractérisé en ce que ledit tube vecteur (V1) comporte une longueur active (L1) formée par ledit tube métallique (23).

5. Dispositif de traitement selon la revendication 4, caractérisé en ce que ladite longueur active (L1) est indépendante de la longueur (L3) de ladite sonde unipolaire (S1, S2).

6. Dispositif de traitement selon l'une des revendications 4 ou 5, caractérisé en ce que ladite longueur active (L1) est limitée par au moins un surgainage (16) dudit tube métallique (23), ledit surgainage (16) étant électriquement isolant.

7. Dispositif de traitement selon la revendication 1, caractérisé en ce qu'il comporte en outre au moins un second générateur (G2), synchronisé en fréquence avec le premier générateur (G1), et relié à une troisième et quatrième sonde unipolaire (S3, S4) coopérant avec les deux premières sondes (S1, S2) pour chauffer ladite zone (8) à traiter.

8. Dispositif de traitement selon la revendication 7, caractérisé en ce qu'il comporte des moyens (41, 42) pour modifier la distribution dudit champ électrique (I1, ..., In) sans modifier l'implantation desdites sondes (S1, S2, S3, S4).

9. Dispositif de traitement selon la revendication 8, caractérisé en ce que au moins un desdits générateurs (G1, G2) comporte des moyens de commutation (41, 42) pour inverser la polarité (+, -) appliquée aux deux sondes unipolaires (S1-S2, S3-S4) auxquelles il est relié, afin de modifier la distribution du champ électrique auquel est soumis ladite zone (8) à traiter.

10. Dispositif de traitement selon l'une des revendications 7 ou 8, ou 9, caractérisé en ce qu'il comporte en outre un oscillateur principal (30) relié auxdits premier et second générateurs (G1, G2) pour les faire fonctionner à une même fréquence.

## Claims

1. A device for treatment by hyperthermy, comprising a generator (G1) supplying alternating electric power, the said electric power being applied, in accordance with an electric field ($I_1$...., $I_n$), to a zone (8) to be treated of a patient by means of two unipolar electrodes (S1 and S2) connected to the said generator (G1), at least one of the said unipolar electrodes (S1 and S2) being of the implantable unipolar probe type, and comprising furthermore at least one vector tube (V1) adapted to be implanted permanently in the zone (8) to be treated, and to contain the said unipolar probes (S1 and S2) during a treatment sitting, the said vector tube (V1) having at least one open tubular extremity (17 and 18), formed of an electrically insulating material, and prolonged by a metallic tube (23), the said metallic tube (23) being adapted to be in contact with the zone (8) to be treated and to contain at least partially the said unipolar probe (S1 and S2), the said tubular extremity (17 and 18) being adapted to be situated at the level of the skin, the said unipolar probe (S1 and S2) being introduced through the said tubular extremity (17 and 18) in the said metallic tube (23) with which it is put into electrical contact.

2. The treatment device as claimed in claim 1, characterized in that the said tubular extremities (17 and 18) are fashioned of a supple material.

3. The treatment device as claimed in the preceding claim, characterized in that the said tubular extremities (17 and 18) are fashioned of PTEF.

4. The treatment device as claimed in any one of the preceding claims, characterized in that the said vector tube (V1) has an active length C /1 L1) formed by the said metallic tube (23).

5. The treatment device as claimed in claim 4, characterized in that the said active length (L1) is independent of the length (L3) of the said unipolar probe (S1 and S2).

6. The treatment device as claimed in any one of the preceding claims 4 and 5, characterized in that the said active length (L1) is limited by at least one over-sheath (16) of the said metallic tube (23), the said over-sheath (16) being electrically insulating.

7. The treatment device as claimed in claim 1, characterized in that it furthermore comprises a second generator (G2), synchronized in frequency with the first generator (G1), and connected with a third and a fourth unipolar probe (S3 and S4) and cooperating with the two first probes (S1 and S2) in order to heat the said zone (8) to be treated.

8. The treatment device as claimed in claim 7, characterized in that it comprises means (41 and 42) in order to modify the distribution of the said electric field ($I_1$, ..., $I_n$) without modifying the implantation of the said probes (S1, S2, S3 and S4).

9. The treatment device as claimed in claim 8, characterized in that at least one of the said generators (G1 and G2) comprises switching means (41 and 42) in order to invert the polarity (+ and −) applied to the two unipolar probes (S1−S2 and S3−S4) with which it is connected in order to modify the distribution of the electrical field to which the said zone to be treated (8) is submitted.

10. The treatment device as claimed in any one of the claims 7 through 9, characterized in that it furthermore comprises a principal oscillator (30) connected with the said first and second generators (G1 and G2) in order to cause them to function at an identical frequency.

**Patentansprüche**

1. Hyperthermie-Behandlungsvorrichtung, mit einem Generator (G1), der eine elektrische alternierende Energie abgibt, wobei diese elektrische Energie als elektrisches Feld ($I_1$, ..., $I_n$) an einer zu behandelnden Zone (8) eines Patienten mittels zweier einpoliger Elektroden (S1, S2) angelegt wird, welche mit dem Generator (G1) verbunden sind, wobei wenigstens eine dieser einpoligen Elektroden (S1, S2) vom Typ einer implantierbaren einpoligen Sonde ist, ferner mit wenigstens einem Vektor-Rohr (V1), das dazu bestimmt ist, in der zu behandelnden Zone (8) dauerhaft implantiert zu sein und eine dieser einpoligen Sonden (S1, S2) während einer Behandlungssitzung zu enthalten, wobei das Vektor-Rohr (V1) wenigstens ein offenes Rohrende (17, 18) umfaßt, das aus einem elektrisch isolierenden Material besteht und durch ein Metallrohr (23) verlängert ist, wobei dieses Metallrohr (23) dazu bestimmt ist, die zu behandelnde Zone (8) zu berühren und wenigstens teilweise die einpolige Sonde (S1, S2) zu enthalten, wobei das Rohrende (17, 18) dazu bestimmt ist, auf Höhe der Haut gelegen zu sein, und wobei die einpolige Sonde (S1, S2) über das Rohrende (17, 18) in das Metallrohr (23), mit welchem sie elektrisch verbunden ist, eingesteckt ist.

2. Behandlungsvorrichtung nach Anspruch 1, dadurch gekennzeichnet, daß die Rohrenden (17, 18) aus einem biegsamen Material bestehen.

3. Behandlungsvorrichtung nach dem vorstehenden Anspruch, dadurch gekennzeichnet, daß die Rohrenden (17, 18) aus Polytetrafluoräthylen bestehen.

4. Behandlungsvorrichtung nach einem der vorstehenden Ansprüche, dadurch gekennzeichnet, daß das Vektor-Rohr (V1) eine aktive Länge (L1), die durch das Metallrohr (23) gebildet ist, aufweist.

5. Behandlungsvorrichtung nach Anspruch 4, dadurch gekennzeichnet, daß die aktive Länge (L1) von der Länge (L3) der unipoligen Sonde (S1, S2) unabhängig ist.

6. Behandlungsvorrichtung nach Anspruch 4 oder 5, dadurch gekennzeichnet, daß die aktive Länge (L1) durch wenigstens eine Abdeckung (16) des Metallrohrs (23) begrenzt wird, wobei diese Abdeckung (16) elektrisch isoliert ist.

7. Behandlungsvorrichtung nach Anspruch 1, dadurch gekennzeichnet, daß sie ferner wenigstens einen zweiten Generator (G2), der mit dem ersten Generator (G1) auf Frequenz abgestimmt ist, umfaßt, und mit einer dritten einpoligen Sonde (S3) sowie mit einer vierten einpoligen Sonde (S4) verbunden ist, die mit den beiden ersten Sonden (S1, S2) zusammenwirken, um die zu behandelnde Zone (8) zu erwärmen.

8. Behandlungsvorrichtung nach Anspruch 7, dadurch gekennzeichnet, daß es Mittel (41, 42) umfaßt, um die Verteilung des elektrischen Feldes ($I_1$, ..., $I_n$) zu verändern, ohne die Implantation der Sonden (S1, S2, S3, S4) zu verändern.

9. Behandlungsvorrichtung nach Anspruch 8, dadurch gekennzeichnet, daß wenigstens einer der Generatoren (G1, G2) Umschaltmittel (41, 42) umfaßt, um die Polarität (+, −) umzupolen, die an die beiden einpoligen Sonden (S1–S2, S3–S4), mit denen er verbunden ist, angelegt wird, damit die Verteilung des elektrischen Feldes, welchem die zu behandelnde Zone (8) ausgesetzt ist, verändert wird.

10. Behandlungsvorrichtung nach Anspruch 7, 8 oder 9, dadurch gekennzeichnet, daß sie ferner einen Hauptoszillator (30), der mit dem ersten Generator (G1) sowie mit dem zweiten Generator (G2) verbunden ist, umfaßt, damit sie auf einer selben Frequenz arbeiten.

FIG_1

GENERATEUR

$G_1$

FIG_2

# FIG_3

# FIG_4